# EUROPÄISCHE PATENTSCHRIFT

(11) **EP 2 683 454 B1**
(45) Veröffentlichungstag und Bekanntmachung des Hinweises auf die Patenterteilung: **12.09.2018**
(21) Anmeldenummer: 11804643.2
(22) Anmeldetag: 09.12.2011
(51) Int. Cl.: C07C 51/44, B01D 3/32

(54) **DESTILLATIONSKOLONNE UND VERFAHREN ZUR DESTILLATION VON ACRYLSÄURE**
DISTILLATION COLUMN AND METHOD FOR DISTILLING ACRYLIC ACID
COLONNE DE DISTILLATION ET PROCÉDÉ DE DISTILLATION D'ACIDE ACRYLIQUE

(30) Priorität: 10.03.2011 DE 102011013561
(43) Veröffentlichungstag der Anmeldung: 15.01.2014
(73) Patentinhaber: Air Liquide Global E&C Solutions Germany GmbH, 60439 Frankfurt am Main (DE)
(72) Erfinder: BAUER, Jochen, 45147 Essen (DE); CASTILLO-WELTER, Frank, 61381 Friedrichsdorf (DE); KREICH, Markus, Dr., 64853 Otzberg (DE); KIRSTEN, Klaus, 55130 Mainz (DE); STEDEN, Christoph, 61440 Oberursel (DE); WALTER, Dominic, 64289 Darmstadt (DE)
(74) Vertreter: Keil & Schaafhausen Patent- und Rechtsanwälte PartGmbB
(86) Internationale Anmeldenummer: PCT/EP2011/006199
(87) Internationale Veröffentlichungsnummer: WO 2012/119629

(56) Entgegenhaltungen:
- WO-A1-98/13117
- DE-A1- 19 631 332
- US-A- 3 766 021
- US-A1- 2006 249 365

## Beschreibung

### Gebiet der Erfindung

Die Erfindung betrifft eine Destillationskolonne mit außen liegendem Umlaufverdampfer, die sich insbesondere zur Durchführung von Destillationsprozessen eignet, bei denen im Kolonnensumpf feste oder gelartige Bestandteile auftreten..Außerdem betrifft diese Erfindung ein Verfahren zur Destillation von Acrylsäure unter Verwendung einer erfindungsgemäßen Destillationskolonne.

Die Bezeichnung Destillationskolonne wird im Folgenden als Oberbegriff verwendet, der auch die Bezeichnung Rektifikationskolonne sowie einstufige Verfahren zur Verdampfungstrennung, wie die sogenannte Flash-Destillation, einschließt.

### Stand der Technik

Destillationskolonnen mit außen liegendem Umlaufverdampfer sind bekannt, sie werden z. B. grundsätzlich beschrieben in dem Buch: Technische Chemie, 2006 Wiley-VCH Verlag GmbH & Co KGaA, Seite 338, Abb. 9.55. In den darin dargestellten Fällen geht die Saugleitung des Verdampferumlaufs von der tiefsten Stelle des Sumpfraums aus und der Teil des Sumpfprodukts, der zur Weiterbehandlung außerhalb der Kolonne vorgesehen ist, wird aus dem Verdampferumlauf abgezogen.

Die Anmeldeschrift US 2006/0249365 A1 zeigt mehrere Alternativen für die Anbindung des Verdampferumlaufs an den Kolonnensumpf. Dabei wird gezeigt, dass die Umlaufleitung entweder an die Auslassleitung für das Sumpfprodukt oder an die Wand, im unteren Teil des Sumpfraums angeschlossen sein kann. Es wird auch ein euterartiger Auslauf des Sumpfraums gezeigt, wobei die Umlaufleitung an diesen Auslauf angebunden ist. Diese Schrift ist mit der Behandlung leicht polymerisierbarer Flüssigkeit befasst und dem Problem von Polymerablagerungen auf einem Leitblech, das oberhalb der Eintrittsöffnung des Verdampferumlaufs in den Kolonnenboden installiert ist. Der Schrift ist aber nichts darüber zu entnehmen, wie verhindert wird, dass im Sumpfprodukt enthaltene Teilchen in den Verdampfumlauf gelangen.

Die Anmeldeschrift DE 196 31 332 A1 befasst sich mit einem Verfahren zum Entfernen störender schwer siedender oder fester Komponenten aus einem Lösemittelkreislauf. Dabei wird das beladene Lösemittel einer Regeneriersäule aufgegeben, die leichtsiedenden Verunreinigungen werden als Kopfprodukt aus der Säule abgelassen, Das Sumpfprodukt stellt das regenerierte Lösemittel dar. Um Feststoffe, Salze und Hochsieder aus dem Sumpfprodukt abzutrennen, wird ein Teil davon in eine, vom Sumpfraum abgetrennte Auffangkammer geleitet. Die in der Auffangkammer befindliche Flüssigkeit wird über einen Verdampferumlauf eingedampft. Der dabei gebildete Dampf wird als Strippdampf in die Regenerierungssäule geleitet. Wenn der gewünschte Eindampfungsgrad erreicht ist, wird die Auffangkammer entleert und anschließend wieder mit Sumpfprodukt befüllt. Die Saugleitung des Verdampferumlaufs ist dabei an die Unterseite der Auffangkammer angeschlossen, d.h. es sind hier keine Maßnahmen getroffen, um den Eintritt von ausgefällten Teilchen in den Verdampferumlauf zu verhindern.

Die Patentschrift US 3,766,021 zeigt eine Destillationskolonne mit außen liegendem Umlaufverdampfer. Vom übrigen Sumpfraum dieser Kolonne ist ein Sedimentationsraum abgetrennt, aus dessen oberen, an Feststoffen abgereicherten Bereich, die Flüssigkeit für den Verdampferumlauf abgesaugt wird. Der Zufluss aus dem Sumpfraum in diese Sedimentationszone erfolgt durch Überfließen der wehrartigen Abtrennwand zwischen Sumpfraum und Sedimentationszone. Es ist zu erwarten, dass durch diese Art des Zuflusses die Flüssigkeit in der Sedimentationszone aufgewühlt wird und damit die Abtrennwirkung der Sedimentationszone beeinträchtigt ist.

Aus EP 1 095 685 B1 ist auch die Möglichkeit bekannt, am Boden des Kolonnensumpfs je einen separaten Auslass für den auszutragenden und für den über den Umlaufverdampfer zirkulierenden Teil des Sumpfprodukts zu installieren.

Beide Varianten haben aber den Nachteil, dass bei Destillationsprozessen, bei denen im Sumpfprodukt feste oder gelartige Bestandteile, deren Dichte über der des Sumpfprodukts liegt, vorhanden sind, diese auf den Boden des Sumpfes absinken, in den Verdampferumlauf gelangen und dabei durch Verschmutzung, insbesondere von Wärmetauscherflächen, zu Betriebsstörungen führen können.

Aus EP 1 095 685 B1 ist bekannt, dass durch Zugabe chemischer Mittel versucht werden kann, die Bildung von festen Teilen im Sumpfprodukt zu verhindern. Nachteilig daran ist aber, neben den Kosten für den chemischen Zusatz, dass dadurch die Qualität des Sumpfprodukts ungewollt verändert wird.

Als alternative Methode schlägt EP 1 095 685 B1 vor, durch Einhaltung bestimmter geometrischer Größenverhältnisse im Bodenablaufbereich der Destillationskolonne, die Verweilzeit des Sumpfprodukts im Kolonnensumpf zu begrenzen und damit die Bildung von festen Bestandteilen, z.B. durch Polymerisation, zu verhindern. Nachteilig an dieser Methode ist, dass sie lediglich die Bildung von festen Teilchen im Kolonnensumpf beeinflusst, aber eine Teilchenbildung im oberen Teil der Kolonne oder die Einschleusung von schon vor der Kolonne gebildeten Teilchen nicht verhindern kann.

Es bestand daher die Aufgabe, eine Destillationskolonne mit außen liegendem Umlaufverdampfer zur Verfügung zu stellen, die, ohne einen erhöhten Reinigungsaufwand zu erfordern, auch für Destillationsprozesse eingesetzt werden kann, bei denen sich feste und/oder gelartige Teile im Sumpfprodukt befinden.

### Beschreibung der Erfindung

Die Aufgabe wurde im Wesentlichen mit der Gesamtheit der Merkmale des Anspruchs 1 dadurch gelöst, dass im Kolonnensumpf eine Sedimentationszone eingerichtet wurde. Die Sedimentationszone ist durch eine Wand vom übrigen Sumpfraum abgetrennt, aber an ihrem unteren Ende zum Sumpfraum hin offen, sodass Sumpfprodukt von unten her der Sedimentationszone zufließen kann. Aus dem oberen, von Feststoff- und/oder Gelpartikeln bzw. Flüssigkeitstropfen geklärtem Bereich der Sedimentationszone, wird der für den Verdampferumlauf vorgesehene Teil des Sumpfprodukts abgezogen und der zur Weiterbehandlung außerhalb der Kolonne vorgesehene Teil des Sumpfprodukts wird an der tiefsten Stelle des Kolonnensumpfs abgezogen.

Sedimentationszonen sind z.B. aus der Konstruktion von Kristallisationsverdampfern bekannt, wie sie z.B. in der Schrift US 3,873,275 beschrieben sind.

Außerdem umfasst diese Erfindung die Verwendung der erfindungsgemäßen Destillationskolonne zur Destillation von Acrylsäure, bei der Rohacrylsäure, die noch unerwünschte Anteile an Acrylsäure-Oligomeren enthält, am Kolonnenkopf als oligomerenfreie Reinacrylsäure erhalten wird. Eine besonders vorteilhafte Ausgestaltung der Destillationskolonne für diese Verwendung sieht vor, dass eine Destillationskolonne gemäß Anspruch 1 verwendet wird, wobei der Verdampferumlauf (5') 20 bis 200, bevorzugt 40 bis 120, m³/(h*m²) und die Größe des für die Weiterbehandlung aus dem Sumpf abgezogenen Stroms (12") 0,1 bis 5, bevorzugt 0,2 bis 3, m³/(h*m²), jeweils bezogen auf die innere Querschnittsfläche der Kolonne, beträgt, wobei das Verhältnis der Durchmesser der Kolonne (1") und des Sumpfes (2") 1:0,1 bis 0,9, bevorzugt 1:0,15 bis 0,6, beträgt und wobei die aufwärtsgerichtete Strömungsgeschwindigkeit in der Sedimentationszone (3") 0,05 bis 1, bevorzugt 0,1 bis 0,6, m/s beträgt. Bei Einhaltung der genannten Parameter wird das Ziel der Erfindung, einen möglichst geringen Anteil an ausgefallenen Feststoff- oder Gelpartikel im Verdampferumlauf zu erhalten, am besten erreicht. Dies gilt insbesondere bei der Verwendung der erfindungsgemäßen Destillationskolonne zur Destillation von Acrylsäure; bei dieser Trennoperationen fallen im Kolonnensumpf insbesondere Partikel aus Acrylsäure-Polymerisat an. Bei Ausgestaltung der Destillationskolonne innerhalb der in Anspruch 3 gelehrten Parameterbereiche wird eine gute Partikelabtrennung bei gleichzeitig hoher Trennleistung hinsichtlich der Reinacrylsäure erzielt. Andere Ausgestaltungen der Destillationskolonne außerhalb der Merkmale des Anspruchs 3 liefern weniger zufriedenstellende Trennergebnisse hinsichtlich der Partikelabtrennung. Das Einziehen des Kolonnensumpfes, d.h. die Ausführung des Kolonnensumpfes mit einem kleineren Durchmesser als die Kolonne, hat sich bei der Acrylsäuredestillation bewährt, da dadurch eine genauere Messung der im Sumpf vorhandenen Flüssigkeitsmenge möglich ist.

### Ausführungsbeispiele:

Weiterbildungen, Vorteile und Anwendungsmöglichkeiten der Erfindung ergeben sich auch aus der nachfolgenden Beschreibung von Ausführungsbeispielen und den Zeichnungen. Dabei bilden alle beschriebenen und/oder bildlich dargestellten Merkmale für sich oder in beliebiger Kombination die Erfindung, unabhängig von ihrer Zusammenfassung in den Ansprüchen oder deren Rückbeziehung.

Anhand der Zeichnung, Fig. 1 und 2 wird beispielhaft jeweils eine erfindungsgemäße Destillationskolonne beschrieben. Fig. 3 zeigt den unteren Teil einer Destillationskolonne mit eingezogenem Sumpf, wie sie üblicherweise zur Acrylsäuredestillation verwendet wird.

Die Destillationskolonne (A, A', A") besteht im Wesentlichen aus der Kolonne (1, 1', 1") und dem Kolonnensumpf (2, 2', 2"). Der Kolonnensumpf ist mit der Sedimentationszone (3, 3', 3") ausgestattet. Die Gestaltung der Sedimentationszone hängt von den Parametern des jeweiligen Verfahrens ab, für das die Kolonne eingesetzt wird. Hierbei werden die Größe der Stoffströme, die Materialeigenschaften der auftretenden Stoffe und die am Aufstellungsort der Kolonne vorhandenen Platzverhältnisse zu berücksichtigen sein.

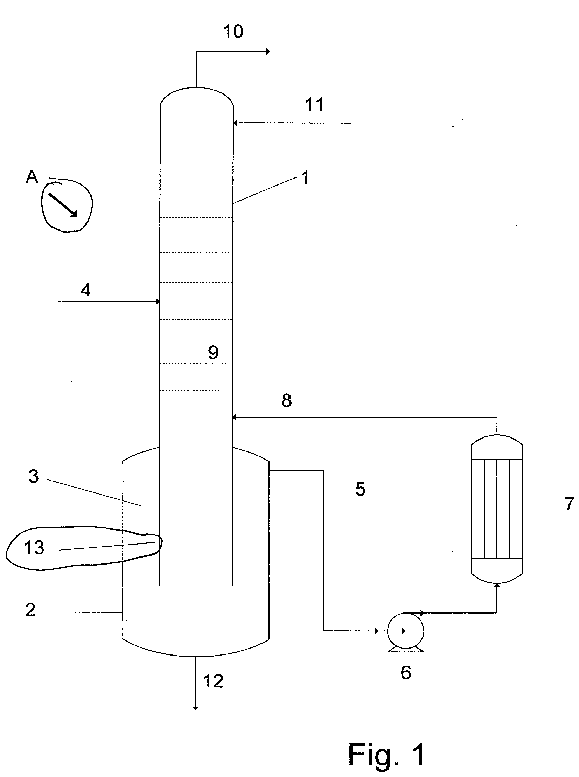

Fig. 1 zeigt eine über den Querschnitt der Kolonne hinausragende Sedimentationszone. Diese Bauart ist zu bevorzugen, wenn die Sedimentation der Partikel im Sumpfprodukt nur langsam erfolgt und darum eine große Verweilzeit in der Sedimentationszone benötigt wird.

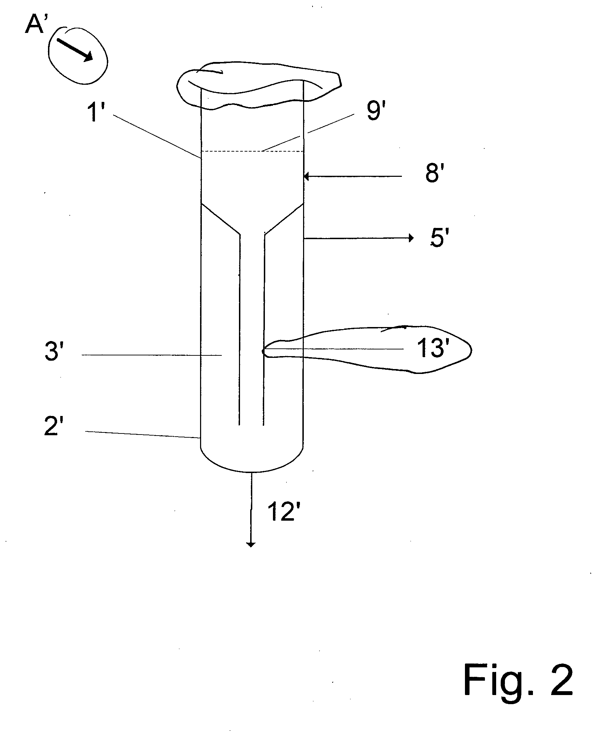

Fig. 2 zeigt ein Ausführungsbeispiel bei dem die Sedimentationszone in den Querschnitt der Kolonne hineingelegt ist. Diese Bauart ist zu bevorzugen, wenn die Platzverhältnisse beschränkt sind und nicht so große Verweilzeiten in der Sedimentationszone erforderlich sind.
   Über Rohrleitung (4) wird das zutrennende Gemisch in die Kolonne eingefüllt. Über Rohrleitung (5, 5', 5") wird Sumpfprodukt aus dem oberen Teil der Sedimentationszone (3, 3', 3") in den Verdampferkreislauf abgezogen, von der Pumpe (6) durch den Verdampfer (7) gefördert und über Rohrleitung (8, 8', 8"), unterhalb der Austauschböden (9, 9') wieder in die Kolonne (1, 1', 1") gefördert. Über Rohrleitung (10) verlässt das Destillat die Kolonne (1), über Rohrleitung (11) wird der Rücklauf des kondensierten Destillats vom, nicht dargestellten, außenliegenden Kondensator zurückgeführt. Am Boden des Kolonnensumpfs wird über Rohrleitung (12, 12', 12") mit Feststoffteilchen beladenes Sumpfprodukt zur weiteren Behandlung abgezogen.

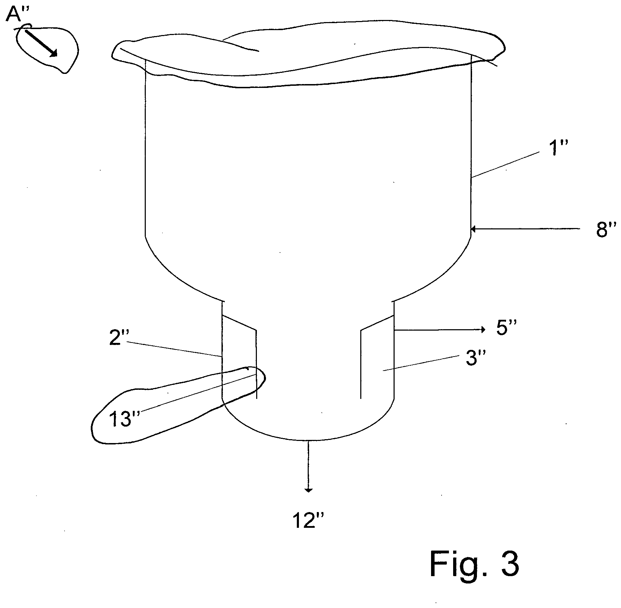

Fig. 3 zeigt ein Ausfühungsbeispiel der erfindungsgemäßen Destillationskolonne wie sie für die Destillation von Acrylsäure üblich ist. Hierbei ist der Sumpf (2") eingezogen, d.h. der Sumpf (2") ist mit einem kleineren Durchmesser als die Kolonne (1") ausgeführt. Dadurch führen Schwankungen den Volumens der im Sumpf befindlichen Flüssigkeitsmenge zu größeren und damit leichter messbaren Änderungen des Pegelstandes. Über Leitung (5") wird aus der Sedimentationszone (3") von Oligomenren geklärtes Sumpfprodukt zum, nicht dargestellten, Umlaufverdampfer abgezogen und über Leitung (8") wieder in die Destillationskolonne (A") eingespeist. Über Leitung (12") wird mit Oligomeren belastetes Sumpfprodukt aus der Destillationskolonne (A") abgezogen und der weiteren Behandlung zugeführt.

### Bezugszeichen:

- (1, 1', 1"): Kolonne
- (2, 2', 2"): Sumpf
- (3, 3', 3"): Sedimentationszone
- (4): Zufuhr Edukt
- (5, 5', 5"): Entnahmeleitung Sumpfprodukt zum Verdampferkreislauf
- (6): Pumpe
- (7): Verdampfer
- (8, 8', 8"): Zufuhrleitung aus dem Verdampferkreislauf zur Kolonne
- (9, 9'): Austauschböden
- (10): Ableitung Destillat
- (11): Rücklauf Kondensat
- (12, 12', 12"): Ableitung Sumpfprodukt
- (13, 13', 13"): Wand
- A, A', A": Destillationskolonne

## Patentansprüche

1. Destillationskolonne (1) mit einem Sumpfraum (2) und einer davon durch eine Wand (13) abgetrennten, aber in hydraulischer Verbindung stehenden Sedimentationszone (3) und mit einem außen liegenden Umlaufverdampfer, (7) zur Durchführung eines Destillationsverfahrens, bei dem im Sumpfprodukt feste oder gelartige Partikel oder Flüssigkeitstropfen mit höherer Dichte als das Sumpfprodukt vorhanden sind,
wobei die Destillationskolonne (1) jeweils einen, an der tiefsten Stelle des Sumpfraums (2) gelegenen, Auslass (12) für den Weiterbehandlung vorgesehenen Teil des Sumpfprodukts und einen im oberen Bereich der Sedimentationszone (3) gelegenen Auslass (5) für den für den Verdampferumlauf vorgesehenen Teil des Sumpfprodukts umfasst, **dadurch gekennzeichnet, dass** die Sedimentationszone (3) an ihrer Unterseite zum Sumpfraum (2) hin offen ist.

2. Verwendung der Destillationskolonne gemäß Anspruch 1 zur Destillation von Acrylsäure.

3. Verwendung nach Anspruch 2, wobei der Verdampferumlauf (5') 20 bis 200, bevorzugt 40 bis 120, m³/(h*m²) und die Größe des für die Weiterbehandlung aus dem Sumpf abgezogenen Stroms (12") 0,1 bis 5, bevorzugt 0,2 bis 3, m³/(h*m²), jeweils bezogen auf die innere Querschnittsfläche der Kolonne, wobei das Verhältnis der Durchmesser der Kolonne (1") und Sumpf (2") 1:0,1 bis 0,9, bevorzugt 1:0,15 bis 0,6, und wobei die aufwärtsgerichtete Strömungsgeschwindigkeit in der Sedimentationszone (3") 0,05 bis 1, bevorzugt 0,1 bis 0,6, m/s beträgt.

## Claims

1. Distillation column (1) having a bottom space (2) and a sedimentation zone (3) separated therefrom by a wall (13) but hydraulically connected thereto and having an external circulation evaporator (7), for performance of a distillation process in which solid or gelated particles or liquid droplets having higher density than the bottom product are present in the bottom product, where the distillation column (1) in each case comprises an outlet (12) at the lowest point in the bottom space (2) for the portion of the bottom product intended for further treatment and an outlet (5) in the upper region of the sedimentation zone (3) for the portion of the bottom product intended for the evaporator circuit, **characterized in that** the sedimentation zone (3) is open at its lower end toward the bottom space (2).

2. Use of the distillation column according to Claim 1 for distillation of acrylic acid.

3. Use according to Claim 2, where the evaporator circulation rate (5') is 20 to 200, preferably 40 to 120, m³/(h*m²) and the size of the stream (12") drawn off from the bottom for the further treatment is 0.1 to 5, preferably 0.2 to 3, m³/(h*m²), based in each case on the internal cross section area of the column, where the ratio of the diameter of the column (1") and bottom (2") is 1:0.1 to 0.9, preferably 1:0.15 to 0.6, and where the upward flow rate in the sedimentation zone (3") is 0.05 to 1, preferably 0.1 to 0.6, m/s.

## Revendications

1. Colonne de distillation (1) comprenant un espace de fond (2) et une zone de sédimentation (3) séparée de celui-ci par une paroi (13), mais en liaison hydraulique avec celui-ci, et un évaporateur à circulation (7) situé à l'extérieur, pour mettre en oeuvre un procédé de distillation, dans lequel des particules solides ou sous forme de gel ou des gouttes de liquide de plus grande densité que le produit de fond sont présentes dans le produit de fond, la colonne de distillation (1) comprenant à chaque fois une sortie (12) placée à l'endroit le plus profond de l'espace de fond (2) pour la partie du produit de fond prévue pour le post-traitement, et une sortie (5) placée dans la région supérieure de la zone de sédimentation (3) pour la partie du produit de fond prévue pour la circulation de l'évaporateur, **caractérisée en ce que** la zone de sédimentation (3) est ouverte au niveau de son côté inférieur vers l'espace de fond (2) .

2. Utilisation de la colonne de distillation selon la revendication 1 pour la distillation d'acide acrylique.

3. Utilisation selon la revendication 2, dans laquelle la circulation de l'évaporateur (5') est de 20 à 200, de préférence de 40 à 120, m³/(h*m²) et la valeur du courant (12") soutiré du fond pour le post-traitement est de 0,1 à 5, de préférence de 0,2 à 3, m³/(h*m²), à chaque fois rapporté à la surface en section transversale interne de la colonne, le rapport du diamètre de la colonne (1") et du fond (2") étant de 1:01 à 0,9, de préférence de 1:0, 15 à 0,6, et la vitesse d'écoulement orientée vers le haut dans la zone de sédimentation (3") étant de 0,05 à 1, de préférence de 0,1 à 0,6 m/s.
